# EUROPEAN PATENT SPECIFICATION

(11) **EP 2 376 627 B1**
(45) Date of publication and mention of the grant of the patent: **15.03.2017**
(21) Application number: 09795527.2
(22) Date of filing: 16.12.2009
(51) Int. Cl.: A61M 1/34, A61K 35/12, C12N 5/071

(54) **IMPROVEMENTS IN OR RELATING TO GROWING CELLS**
VERBESSERUNGEN FÜR UND DAMIT ZUSAMMENHENGEND DAS WACHSTUM VON ZELLEN
AMELIORATIONS APPORTEES DANS ET A PROPOS DE LA CROISSANCE CELLULAIRE

(30) Priority: 17.12.2008 GB 0822961
(43) Date of publication of application: 19.10.2011
(73) Proprietor: UCL Business PLC, London W1T 4TP (GB)
(72) Inventor: SELDEN, Clare, London W1 4TP (GB); ERRO, Eloy, London W1T 4TP (GB)
(74) Representative: Hammond, David Matthew
(86) International application number: PCT/GB2009/051724
(87) International publication number: WO 2010/070342

(56) References cited:
- US-A1- 2002 168 758
- KINASIEWICZ ET AL: "Culture of C3A Cells in Alginate Beads for Fluidized Bed Bioartificial Liver" TRANSPLANTATION PROCEEDINGS, ORLANDO, FL, US, vol. 39, no. 9, 20 November 2007 (2007-11-20), pages 2911-2913, XP022386965 ISSN: 0041-1345
- DAVID B ET AL: "In vitro assessment of encapsulated C3A hepatocytes functions in a fluidized bed bioreactor" BIOTECHNOLOGY PROGRESS JULY/AUGUST 2004 AMERICAN CHEMICAL SOCIETY US, vol. 20, no. 4, July 2004 (2004-07), pages 1204-1212, XP002569684
- CHAN C ET AL: "APPLICATION OF MULTIVARIATE ANALYSIS TO OPTIMIZE FUNCTION OF CULTURED HEPATOCYTES" BIOTECHNOLOGY PROGRESS, AMERICAN INSTITUTE OF CHEMICAL ENGINEERS, US, vol. 19, no. 2, 1 March 2003 (2003-03-01), pages 580-598, XP008052619 ISSN: 8756-7938
- FIEGEL H.C. ET AL: "Hepatic tissue engineering: from transplantation to customized cell-based liver directed therapies from the laboratory" JOURNAL OF CELLULAR AND MOLECULAR MEDICINE, vol. 12, no. 1, January 2008 (2008-01), pages 56-66, XP002569685 ISSN: 1582-1838

## Description

### TECHNICAL FIELD

The present invention relates to the field of growing cells and more particularly to growing cells to performance competence in a matrix, such as alginate beads. It has particular application in the field of extracorporeal liver perfusion and in the development of a bio-artificial liver.

### BACKGROUND OF THE INVENTION

The applicant, in their earlier, as yet unpublished, patent application GB 0713595.7 disclose a system in which a proliferating cell line (in contrast to primary cells) is seeded into a matrix, optionally with one or more cell density modifiers, where the cells proliferate into a three dimensional mass. The cells, when they reach performance competence, are able to function like a liver since they form a structural body in association with the matrix.

A problem with the methodology described in the earlier application, is that it has not been possible to grow the cells, in a scalable manner, to achieve a cell density with an order of magnitude greater than an order of 10⁶ cell per ml of beads. This means that relatively large volumes (several litres) of the biological component are required to achieve the desirable number of cells for the function required. This in turn poses difficulty in getting nutrients and oxygen to the three dimensional mass of beaded cells (due to the volume).

The applicant has surprisingly discovered that, by supplementing a culture media, with human plasma, in the form of leucocyte depleted fresh frozen plasma, as opposed to foetal calf serum, they have been able to achieve a considerably higher cell density per bead than has hitherto been possible.

This has significant implications in the development of bio-artificial organs since any reduction in the overall volume of the biological component brings forward the prospect of successful commercialisation.

Currently, the sole cure for both acute and chronic liver disease and liver failure is transplantation. However, this treatment is restricted by a lack of donor organs. In the US alone, 17,000 patients are on the transplant waiting list. Many of them die whilst waiting for a transplant. There is therefore an urgent need for a device which can temporarily perform the function of a patient's liver, keeping them alive whilst a suitable donor organ is found, or which provides an environment which ensures the patient does not die whilst the patient's own liver recovers sufficient functionality for the patient's survival.

There are two principal types of liver machines:
- A purely artificial machine; and
- A bio-artificial machine.
Both rely on perfusion of a patient's plasma or blood into an extracorporeal circuit for a period of 6h or more.

Purely artificial systems exist and a number of bio-artificial systems are in development.

Purely artificial systems, such as albumin dialysis, are however unable to replace all the liver functions including:
- Detoxification,
- Biotransformation,
- Synthesis, and
- Storage
and whilst they have proved relatively safe in clinical trials they have not given rise to a significant improvement in patient survival.

The purely artificial systems are solely physical/chemical in nature, and provide a detoxification function by adsorption/exchange on e.g. resin, charcoal, ion exchange columns or albumin, or combinations of these.

In contrast the bio-artificial livers (BALs) contain a biological component, i.e. liver cells, either alone, or in conjunction with an artificial device as a hybrid system. The hypothesis underlying the incorporation of liver cells is that liver function is so complex, comprising multiple synthetic, detoxification, and metabolic pathways, that crude mechanical devices will always be inadequate to replace the range of function desired; furthermore the functions critical to buying time for liver function to recover, have not been fully defined and the use of liver cells allows both defined and undefined functions to be replaced. For the biological component, isolated liver cells or occasionally liver slices are used, and systems have used either human or animal (most often porcine) cells.

The majority of early BALs used hollow fibre cartridges in which cells were separated from plasma or whole blood by a membrane. Pore sizes of the membrane differed between systems, some limited to transfer of molecules <10,000 daltons, some with pore sizes as large as 2 micron.

More recently other configurations have emerged which better address mass transfer limitations. They include:
- An AMC-BAL which contains cells attached to a polyester matrix which is exposed directly to oxygenated plasma, (Flendrig LM, LaSoe JW, Jorning GGA, Steenbeek A, Karlsen OT, Bovee WMMJ, Ladiges NCJJ, TeVelde AA, Chamuleau RAFM. In vitro evaluation of a novel bioreactor based on an integral oxygenator and a spirally wound non-woven polyester matrix for hepatocyte culture as small aggregates. Journal of Hepatology 1998; 26: 1379-1392.)
- A non-woven fabric bioreactor, (Li LJ, Du WB, Zhang YM, Li J, Pan XP, Chen JJ, Cao HC, Chen Y, Chen YM. Evaluation of a bioartificial liver based on a non-woven fabric bioreactor with porcine hepatocytes in pigs. Journal of Hepatology 2006; 44: 317-324.)

- A radial flow bioreactor, (Morsiani E, Brogli M, Galavotti D, Bellini T, Ricci D, Pazzi P, Puviani AC. Long-term expression of highly differentiated functions by isolated porcine hepatocytes perfused in a radial-flow bioreactor. Artif.Organs 2001; 25: 740-748.) and
- The Innsbruck Bioartificial Liver containing hepatocyte aggregates. (Hochleitner B, Hengster P, Duo L, Bucher H, Klima G, Margreiter R. A novel bio-artificial liver with culture of porcine hepatocyte aggregates under simulated microgravity. Artif.Organs 2005; 29: 58-66.)
The above examples all use animal hepatocytes.

Examples of reactors with human cells include those using:
- Primary hepatocytes, (Gerlach JC, Mutig K, Sauer IM, Schrade P, Efimova E, Mieder T, Naumann G, Grunwald A, Pless G, Mas A, Bachmann S, Neuhaus P, Zeilinger K. Use of primary human liver cells originating from discarded grafts in a bioreactor for liver support therapy and the prospects of culturing adult liver stem cells in bioreactors: a morphologic study. Transplantation 2003; 76: 781-786.)
- Hollow fibre cartridges using well-differentiated tumour-derived cell lines such as C3A cells, (Ellis AJ, Hughes RD, Wendon JA, Dunne J, Langley PG, Kelly JH, Gislason GT, Sussman NL, Williams R. Pilot-controlled trial of the extracorporeal liver assist device in acute liver failure. Hepatology 1996; 24: 1446-1451.) and
- A fluidised bed bioreactor with human C3A cells encapsulated at high (about 1 million cells/ml) density into alginate. (David B, Dufresne M, Nagel MD, Legallais C. In vitro assessment of encapsulated C3A hepatocytes functions in a fluidized bed bioreactor. Biotechnol.Prog. 2004; 20: 1204-1212.)

Various groups around the world are working with different biological components including:
- The use of primary cultures of human hepatocytes;
- The use of primary cultures of pig hepatocytes; and
- The use of C3A cells - a proliferating cell line initially derived from a well developed human liver cell tumour.

There are fundamental differences between any system which uses proliferating cell lines and those which use primary cells. Applicant's proliferating cell lines can be seeded singly and multiply, in situ, to form cohesive spheroids over a period of time, dependent on the doubling time of a specific cell type. In contrast, primary cells even if seeded at a very high cell density will not necessarily form close cell to cell contacts and therefore will not necessarily give rise to a true 3-dimensional environment, which is associated with up-regulation of function, as it mimics the in vivo situation.

The applicant's approach has been to use a cell line, and has similarities with the C3A approach which has not yet proved effective in clinical trials. However, the applicant's cell line is different and has some different functional properties. There are also fundamental differences between the housing and initial culture of the cells prior to use.
Previously, C3A cells have been used either in:
- "Hollow fibre cartridge culture configuration", or
- "Uncultured", in a fluidised bed in low occupancy alginate beads.
This is in contrast to the methodology used by the applicant, who uses an uncoated alginate matrix in a fluidised bed bioreactor configuration with preculture of encapsulated cells to performance competence.
The applicants biological component, which comprises human hepatocyte cell lines cultured in a 3-D configuration, has been demonstrated, at lab scale, to provide functional liver capacity on a per cell basis, which approaches that seen in vivo for several of the liver's key functions including:
- Clotting factor synthesis;
- Steroid metabolism; and
- Specified detoxification functions.

Fuller details on the expression of hepatocyte-specific function, pioneered by the applicant, are given below:

Applicant has pioneered (on a laboratory scale) the culture of human hepatocyte-derived cell lines as 3-dimensional (3-D) spheroid colonies in alginate beads, as disclosed in:
- Selden C, Shariat A, McCloskey P, Ryder T, Roberts E, Hodgson H. Three-dimensional in vitro cell culture leads to a marked upregulation of cell function in human hepatocyte cell lines--an important tool for the development of a bioartificial liver machine. Annals Of The New York Academy Of Science 1999; 875: 353-363;
- McCloskey P, Edwards RJ, Tootle R, Selden C, Roberts E, Hodgson HJ. Resistance of three immortalized human hepatocyte cell lines to acetaminophen and N-acetyl-p-benzoquinoneimine toxicity. J Hepatol 1999; 31: 841-851;
- Selden C, Khalil M, Hodgson H. Three dimensional culture upregulates extracellular matrix protein expression in human liver cell lines-a step towards mimicking the liver in vivo? Int J Artif Organs 2000; 23: 774-781;
- Khalil M, Shariat-Panahi A, Tootle R, Ryder T, McCloskey P, Roberts E, Hodgson H, Selden C. Human hepatocyte cell lines proliferating as cohesive spheroid colonies in alginate markedly upregulate both synthetic and detoxificatory liver function. Journal Of Hepatology 2001; 34: 68-77;
- McCloskey P, Tootle R, Selden C, Larsen F, Roberts E, Hodgson HJ. Modulation of hepatocyte function in an immortalized human hepatocyte cell line following exposure to liver-failure plasma. Artif.Organs 2002; 26: 340-348; and
- Coward SM, Selden C, Mantalaris A, Hodgson HJ. Proliferation rates of HepG2 cells encapsulated in alginate are increased in a microgravity environment compared with static cultures. Artif.Organs 2005; 29: 152-158.

The advantages of this system are:
- Cells proliferating in this milieu maintain a near-cuboidal cell architecture;
- They have close cell-to-cell and cell-matrix organisation; and
- They secrete extracellular matrix proteins and a large repertoire of liver specific secreted proteins as exemplified by:
   - Albumin,
   - Prothrombin,
   - Fibrinogen,
   - Alpha-1-antitrypsin, and
   - Alpha-1-acid glycoprotein.
They express many functions at levels equivalent to those of hepatocytes in vivo, e.g. steroid metabolism, glycogen synthesis etc.

The applicant has also shown that some functions are poorly expressed or missing, but can be supplemented. For example they have demonstrated that although HepG2 clones, including the C3A subclone which is the basis of one bio-artificial device, produce urea, this is via a urea-cycle independent mechanism that does not detoxify ammonia. Thus, unmodified, such cells are unlikely to be beneficial in treating the ammonia-dependent encephalopathy of liver failure. Using gene transfer to replace two missing enzymes they have demonstrated restoration of urea production from ammonia in their HepG2 clones. (Mavri-Damelin D, Eaton S, Damelin LH, Rees M, Hodgson HJ, Selden C. Ornithine transcarbamylase and arginase I deficiency are responsible for diminished urea cycle function in the human hepatoblastoma cell line HepG2. Int.J Biochem.Cell Biol. 2006.)

They have also characterised them extensively with respect to liver specific function as disclosed in:
- (Khalil M, Shariat-Panahi A, Tootle R, Ryder T, McCloskey P, Roberts E, Hodgson H, Selden C. Human hepatocyte cell lines proliferating as cohesive spheroid colonies in alginate markedly up-regulate both synthetic and detoxificatory liver function. Journal Of Hepatology 2001; 34: 68-77;
- Selden C, Shariat A, McCloskey P, Ryder T, Roberts E, Hodgson H. Three-dimensional in vitro cell culture leads to a marked upregulation of cell function in human hepatocyte cell lines - an important tool for the development of a bioartificial liver machine. Annals Of The New York Academy Of Science 1999; 875: 353-363; and
- Selden C, Khalil M, Hodgson H. Three dimensional culture upregulates extracellular matrix protein expression in human liver cell lines -a step towards mimicking the liver in vivo? Int J Artif Organs 2000; 23: 774-781)
(All referred to previously) and
- LH Damelin, M Kirwan, S Coward, P Collins, IJ Cox, C Selden, HJF Hodgson. Fat-loaded insulin resistant HepG2 cells are resistant to cytokine and pro-oxidant induced damage, but become damage susceptible after down-regulation of AMP-activated kinase. BASL 2005; and
- Selden C, Roberts E, Stamp G, Parker K, Winlove P, Ryder T, Platt H, Hodgson H. Comparison of three solid phase supports for promoting three- dimensional growth and function of human liver cell lines. Artif.Organs 1998; 22: 308-319.

Additionally, in an animal model of fulminant liver failure, they have shown them to exhibit an improvement in clinical and biochemical parameters.
- Rahman TM, Selden C, Khalil M, Diakanov I, Hodgson HJ. Alginate-encapsulated human hepatoblastoma cells in an extracorporeal perfusion system improve some systemic parameters of liver failure in a xenogeneic model. Artif.Organs 2004; 28: 476-482; and
- Rahman TM, Selden AC, Hodgson HJ. A novel model of acetaminophen-induced acute hepatic failure in rabbits. J Surg.Res. 2002; 106: 264-272.

Furthermore, they have demonstrated improved per bead performance by culture in a rotating cell culture system (RCCS) under simulated microgravity conditions:
- Coward SM, Selden C, Mantalaris A, Hodgson HJ. Proliferation rates of HepG2 cells encapsulated in alginate are increased in a microgravity environment compared with static cultures. Artif.Organs 2005; 29: 152-158
   (Referred to previously); and
- Human liver cells in a pilot scale fluidised bed bioreactor maintain performance in human liver failure plasma, making them suitable for a bioartificial liver. Presented at World Congress of Biomechanics, July 29-Augst 4 2006 in Munich, Germany.

They have also tested the performance of this system in normal human plasma and plasma collected from patients with acute liver failure establishing that there is maintained viability and functional performance over 8 hours.
- S.M.Coward, C.Legallais, M.Thomas, F.Tofteng, F.Larsen, H.J.Hodgson, C.Selden. Alginate-encapsulated Hepg2 cells in a pilot-scale fluidised bed bioreactor maintain performance in human liver failure plasma making them suitable for use in a bioartificial liver. Journal of Hepatology 44 [Suppl 2], S53. 2006.

However, the scale up of the biological component, from a laboratory scale size, involving no more than a 70ml volume of alginate beads, provides significant challenges.

Thus, for example, the biological component of the extracorporeal system should be:
- Prepared to appropriate good manufacturing practice (GMP),
- Readily transportable to centres where the patients will be hospitalised,
- Conveniently packaged for storage, transport and charging a perfusion system;
- Movable from the "cell-factory" through to the "clinic", for use in extracorporeal circulation.

US 6,218,182 teaches a tissue engineering bioreactor for growing three dimensional tissue in which cells are seeded onto a mesh. After the tissue has been grown in the bioreactor, it is suggested that it can be frozen and preserved in the bioreactor container itself.

GB 0713595.7 discloses a chamber, in which a biological component can be housed to form, for example, a bio-artificial liver (BAL), which is functionally modular in that it can retain the biological component in a manner which allows it to:
- Proliferate the biological component (in situ);
- Cryopreserve (both freeze and defrost) the biological component (in situ); and
- Be perfused.

The use of a feed supplemented with human plasma to grow a biological component affords substantial benefits over applicants prior art methodology in which the maximum number of performance competent cell spheroids which could be obtained by the method disclosed was 2.5 x 10⁷ cells/ml.

US2002/0168758 discloses the circulation of a fluid through the inner fibres of a bioartifical liver. Suitable fluids include human fresh frozen plasma. It does not however teach growing cells to performance competence using such media.

US6294380 discloses scaling up established functional cells on macroporous carriers. The hepatocytes became confluent under perfusion culture conditions. Upon harvest of cells, the density could reach 10⁷⁻10⁸ cells/ml.

### PRESENT INVENTION

According to a first aspect of the present invention there is provided a biological component (100) comprising:
i. a matrix forming agent (120),
ii. a plurality of cells (110), and
iii. one or more density modifiers (130),
characterized in that, at performance competence, the cells are present in the matrix forming agent at a density of > 3 x10⁷ cells/ml.

Here, "performance competence" is defined as achieving a state in which the cells act in a manner akin to that of functional (liver) cells.

Preferably the cells are present in the matrix forming agent at a density of at least 3 x 10⁷ cells/ml, though 3.5 x 10⁷ cells/ml, 4.0 x 10⁷ cells/ml, 4.5 x 10⁷ cells/ml, 5.0 x 10⁷ cells/ml, 5.5 x 10⁷ cells/ml, 6.0 x 10⁷ cells/ml, 6.5 x 10⁷ cells/ml and 7.0 x 10⁷ cells/ml.

The cells may reach such a cell density in from 8-13 days at which point they exhibit performance competence.

Preferably the cells are a proliferating human cell line exhibiting a hepatocyte phenotype.

The cells are preferably a Hep G2 cell line with or without genetic modification.

Preferably the matrix forming agent is an alginate, more preferably alginate beads. The beads may be any appropriate size, but a diameter of from 300-1200µm has been found to be particularly beneficial.

Achieving a high density of performance competent cells in the beads is a consequence of the methodology employed and also the level of seeding.

According to a second aspect of the present invention there is provided a method of growing a plurality of cells (110) to performance competence in a matrix forming agent (120) containing one or more density modifiers (130) characterized in that the cells are grown in a media supplemented with 2-20% plasma.

More preferably the media is supplemented at a level of 5-15%, most preferably about 10%.

Preferably the plasma is leukocyte depleted fresh frozen human plasma. This may contain anti-clotting agents such as heparin. Where the anti-clotting agent is heparin this may be present in an amount of between 40 -120 units /ml plasma.

Typically the heparin is added at levels of 20u/ml plasma/day with an initial inoculums of 40u/ml.

By utilising the biological component of the invention, grown using the methodology of the invention, it is possible to fill a chamber, with relatively low volumes of the biological component (less than 2 litres), of the type described with reference to GB0713595.7 to produce a bioartifical liver.

The method allows faster cell proliferation (growth to performance competence) and more dense cell packing within the bead without loss of cell function such that the per cell performance remains the same but the per bead performance is significantly enhanced. Typically a bead packing of > 25% is achieved with FFP representing a > 5 fold enhancement over FCS supplementation.

It will be appreciated that the method might be used to grow other mammalian epithelial cells, such as, stem or progenitor cells or other established human epithelial cell lines.

The invention also removes animal components from the system, thus removing the risk of zoonosis. The use of human components is generally considered beneficial to the patient and the increased speed of growth and reduced volumes provide significant economic benefit. However, the greatest benefit arises from the reduction in the volume of biological material needed to fill the chamber.

According to a third aspect of the present invention there is provided a bio-artificial liver (200) comprising a chamber (10) filled with a biological component (100) comprising:
i. a matrix forming agent (120),
ii. a plurality of cells (110), and
iii. one or more density modifiers (130),
characterized in that, at performance competence, the cells are present in the matrix forming agent at a density of at least 3 x10⁷ cells/ml.

The bio-artificial liver (200) may comprise from 3x10¹⁰ to 1x10¹¹ competent cells in a volume of less than 2 litres.

The bio-artificial liver (200) is preferably one as described in GB0713595.7 wherein the chamber comprises a fluid bed support (20), a fluidising inlet (16) and a fluidising outlet (18).

According to a forth aspect of the present invention there is provided a scalable method for proliferating cells seeded in a matrix forming agent containing one or more density modifiers comprising:
a. Placing the cells seeded in the matrix forming agent in a chamber having a fluidised bed, and
b. Growing them to performance competence in a media supplemented with 2-20% plasma.

Preferably the chamber for the biological component of the bio-artificial liver is configured to allow:
- Proliferation of the biological component, in situ;
- Cryopreservation of the biological component, in situ, and
- Perfusion of the biological component, in situ.

Preferably, the chamber is also designed such that it can be sterilised before the biological component is introduced into the chamber.

Preferably, the biological component comprises:
- A cell line, most preferably Hep G2 cells ;
- A matrix forming agent, most preferably alginate beads; and
- A density controlling agent, most preferably glass beads.

The cell line is encapsulated in the matrix forming agent together with the density controlling agent.

The encapsulation serves a number of functions:
- It protects cells during freeze/thawing
- It facilitates easy handling of the biomass
- It allows for perfusion in a chamber or fluidised bed reactor; and
- It provides a matrix allowing the cells to form a three dimensional mass.

That such alginate encapsulated cells may be suitable for scale up has been disclosed by the applicant - Coward et al Poster abstract Dec 4-5 2006 (incorporated by reference).

Preferably the alginate is seeded with 0.5x10⁶ to 5.0x10⁶ cells per ml of alginate, more particularly still from 1.5 x10⁶ to 2.5 x10⁶ cells/ ml of alginate If too few cells are used, they take too long to grow and conversely, if too many are used they won't proliferate.

The chamber serves as a fluidised bed reactor and comprises:
- An inlet and outlet allowing for fluid flow across the chamber; and
- A fluid bed support above which the encapsulated cells can be suspended.

The general concept of using a fluidised bed for perfusion has also been previously disclosed - Legallais et al, Artificial Organs 24(7):519-525. (Incorporated by reference.) It has not, however, been previously suggested that a fluidised bed could beneficially be used in the proliferation stage nor with cell spheroids, only with encapsulated single cell suspensions.

The fluid bed support preferably comprises a plate which is provided with a plurality of spaced holes, of not greater than 200µm, which allow for controlled fluidisation of the bed. The bed has a filter overlaying it which is held in place by e.g. a wire ring. The plate is sealed within the chamber by, for example, an 0-ring.

The chamber is preferably cylindrical and has an aspect ratio (a) = height (h) / diameter (d) of from 10:1 to 1.3:1. This is significant for two reasons: In its upright (vertical) position such an aspect ratio provides an optimum environment (mass/ volume relationship characteristics) for initial cell proliferation and for use, i.e. for transfer of oxygen and nutrients to the cells and for transfer of proteins from the cells to e.g. plasma or culture medium. It also facilitates medium exchange prior to cryopreservation. (Rapid freezing/ defrosting of cells.) In its horizontal position, (particularly when the aspect ratio approaches 10:1) the biological component can be "spread", such that a larger surface area thereof can be brought into contact with the outer wall of the chamber and heat exchange elements, thus facilitating better heating/ cooling (with minimal disruption to fluidisation) due to the laterally displaced position of the heat exchange elements relative to the main chamber volume.

To further address the problem of getting sufficient oxygen to the cells, in a device scaled for human use (typically one of a size of from 1 - 10 litres volume capable of holding 0.5 - 51 of a biological component and which should be capable of allowing for up to a two-fold expansion of volume on fluidisation) the chamber additionally comprised a fluid transport system disposed therein. The fluid transport system has a primary function of transporting gas to the biological component, but may additionally have a secondary function of transporting a heating or cooling fluid. In order to facilitate its primary function it is made of a gas permeable material and is disposed within the chamber in a manner that ensures a detrimental oxygen gradient does not build up, in use, from the inlet to outlet.

In one favoured embodiment the fluid transport system is disposed helically around the inner circumferential walls of the chamber.

In a further preferred embodiment the fluid transport system has a degree of compressibility, such that it can function as a "shock absorber" during cryopreservation, thereby additionally providing a degree of protection to the encapsulated biological component.

The various aspects of the invention are further described, by way of example only, with reference to the following figures in which:
Figs 1 a and 1 b are respectively, a slide comparing FCS grown cells in alginate with FFP grown cells in alginate at day 5 in static culture;
Fig 2 is a graph showing the cell density (millions of cells / well) of FCS grown cells in alginate compared to FFP grown cells in alginate at day 5 in static culture of alginate beads;
Fig 3 is a graph comparing AFP production (mic g AFP) for FFP and FCS grown cells in alginate at day 5 in static culture
Fig 4 is a graph comparing AFP production (million cells/ 24h) for FFP and FCS grown cells in alginate at day 5 in static culture
Figs 5a and 5b are slides showing FFP grown cells at performance competence (7 days) alive and under phase contrast respectively seeded 0.5 x 10⁶/ml at day 0;
Fig 6 is a graph showing the total number of cells/ ml of beads when grown with 10% FFP and 10%FCS respectively;
Fig 7 is a cross sectional view of one embodiment of a chamber according to the invention;
Figs 8a and 8b are phase contrast images of the biological component,
Fig 9a is a cross sectional view of a BAL in the vertical (general use) position;
Fig 9b is a cross sectional view of a BAL in the horizontal (cryopreservation) position; and
Fig 10 shows a BAL in a circuit for in line use.

### DETAILED DESCRIPTION

The benefit of using a media supplemented with 2-20% plasma, particularly fresh frozen plama (FFP) as opposed to foetal calf serum (FCS) is illustrated with reference to the Examples below: Numerical references to the device tie to the subsequent description.

### Example 1.

### Proliferation to performance competence

### Methodology

1. The device (200) is filled with culture medium (40) e.g. alpha MEM, to the level of the filter (26) using a peristaltic pump to deliver medium thereto;
2. Encapsulated cells (100) are introduced into the chamber (10) via a fill port (not shown) in upper plate (12);
3. The remaining volume (v) of the chamber is completely filled with culture medium (40);
4. The BAL (200) is connected to a separate circuit (not shown) that supplies culture medium (40) via a peristaltic pump from the reservoir of a, for example, Celligen Plus bioreactor (New Brunswick Scientific) and returns it to the same reservoir via an inline dissolved oxygen probe;
5. The flow rate used is such that a 1.2 to 2 fold expansion of the packed bed of alginate beads (100) is achieved;
6. An automated bioreactor controller both monitors and controls the temperature, stirring rate, pH, and dissolved oxygen of the culture medium reservoir. This allows set points to be chosen which ensure that medium delivered to the fluidised bed bioreactor is optimal for encapsulated cell growth;
7. Approximately 50% of the volume of culture medium in the circuit is replaced after the first 48-72 hours of culture and from then on every 24 to 48 hours. A feed and bleed system is also available for more subtle control of specific reagents as required.
8. A cassette of immobilised enzymes, in direct contact with the flow path, will be introduced immediately prior to the culture medium in contact with beads that recycle lactate to provide pyruvate (an energy source) and oxygen to the beads. The enzymes to be used will include, but not be limited to, lactate oxidase and catalase.

During proliferation, cytochrome P450 activity may be enhanced by culturing with an inducer, such as, for example dibenzanthracene (at e.g. 6µM) for a time period of e.g. 24-72 hours. This can significantly elevate Cytochrome P450 levels for a period of days following induction (which remains sustainable once the inducer is removed) and can furthermore, on subsequent exposure result in further elevation.

Alginate encapsulated cells (100) can be removed from the chamber (10) during proliferation and are typically maintained for 8 to 12 days in order for encapsulated cells to reach performance competence.

The culture medium was either:
FCS enhanced
   High glucose alpha MEM complete media with (100ul of 1 M CaCl2) enhanced with 10% FCS. or
FFP enhanced
   High glucose alpha MEM complete media with (220ul of 1 M CaCl2) enhanced with 10% FFP

A summary of the conditions used are set out in table 1 below:

**Table 1**

| | FBB16 | FBB 20 |
|---|---|---|
| Cells | Hep G2 | Hep G2 |
| Beads | 200ml | 200ml |
| Media | High glucose alpha MEM complete media with (100ul of 1 M CaCl2) enhanced with 10% FCS. | High glucose alpha MEM complete media with (220ul of 1 M CaCl2) enhanced with 10% FFP |
| Duration of experiment | 9 days | 13 days |
| Media changes on days | 2,5,7 | 2,5,7,9,11,12 |
| Media volume in the circuit | 5.5l | 71 |
| Volume of media changed | 2.75l | 3.5l (1.751 day 12) |
| Chamber | 10cm dia | 15cm dia |
| Bicarbonate | yes | yes |
| Daily added heparin | no | 200U/ml (2.8ml of 5000U/ml stock |
| Fluidisation | 1.5 times | 1.5 times |
| Flow rate | 90ml/min | 390ml/min |

### Results

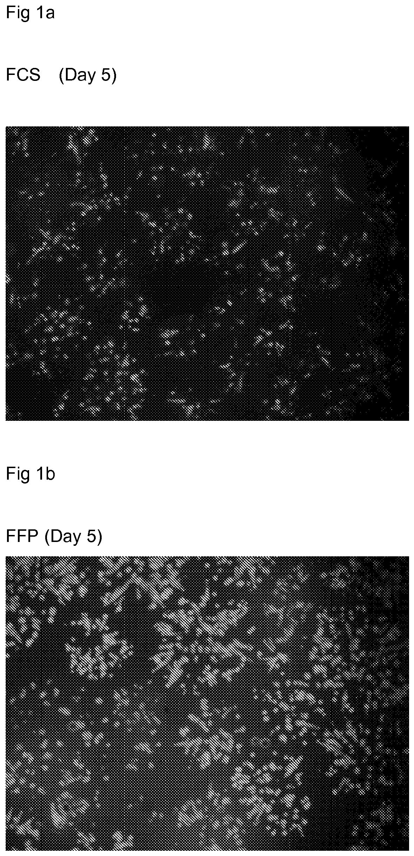

It can be seen by comparison of Fig 1 a and Fig 1 b that at 5 days the FFP grown cells are growing faster, and have a much greater cell density, than those grown in FCS.

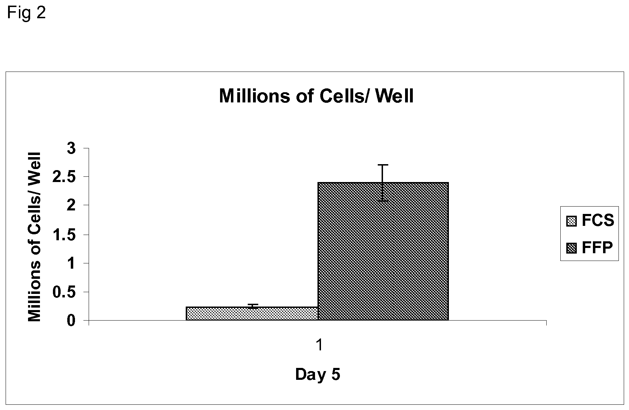

Also, as is apparent from Fig 2, there are some approx 2.5 million cells/ well in the FFP grown media as compared to approx 0.25 million in the FCS grown media (a 10 fold difference).

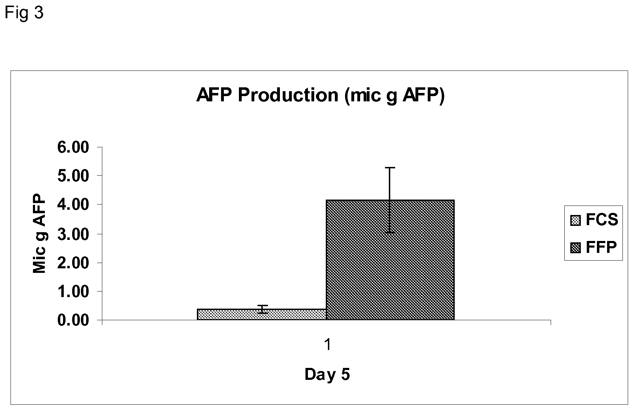

Furthermore, as illustrated in Fig 3 the cells Alpha feto protein levels (AFP). which for the Hep G2 cells in plasma are a good indicator of cellular metabolism/ performance, are significantly higher for the FFP supplemented cells (approx 4.0 mic g/ml vs 0.3 mic g/ml conditioned medium ,again greater than a 10 fold difference.

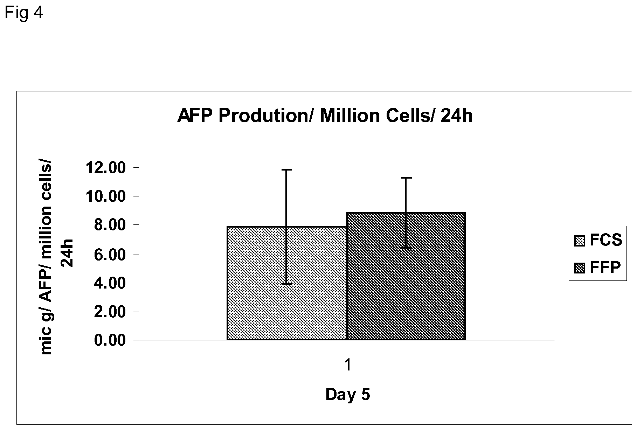

Even allowing for the same number of cells (Fig 4) AFP production was increased slightly after 24 hours in FFP supplemented media.

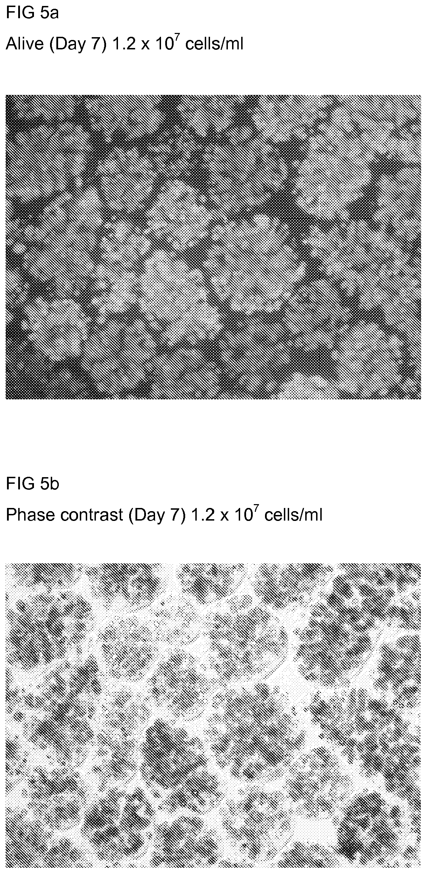

The dense nature of the cells is best illustrated in Figs 5a and 5b which shows cell containing alginate beads after 7 days growth with FFP supplemented culture media in static culture. At this stage they have reached a cell density of 1.2 x 10⁷ cells /ml.

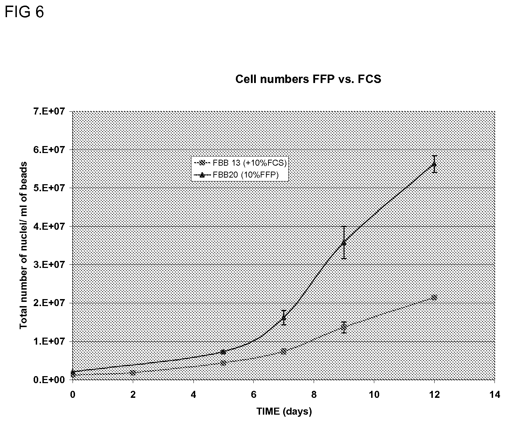

In the fluidised bed bio reactor, in contrast to the static culture, higher cell densities were achieved with both FCS and FFP supplemented media. However there was a very significant increase in the highest cell density achieved using FFP supplemented media. This is illustrated in Table 2 below which results are shown graphically in Fig 6:

**Table 2**

| | **FBB13 cells/ml bead FCS** | **FBB20 cell/ml bead FFP** |
|---|---|---|
| 0 | 1.26E+06 | 2.06E+06 |
| 5 | 4.44E+06 | 7.32E+06 |
| 7 | 8.08E+06 | 1.62E+07 |
| 9 | 1.37E+07 | 3.58E+07 |
| 12 | 2.14E+07 | 5.63E+07 |

The significance of this data will be apparent when one extrapolates cell density with culture time and considers the volume of the biological component necessary to achieve the cell yields that would be necessary to provide something with the functional equivalence with a partially functional liver (at least 15% function) of 1-2 x 10¹¹ cells.

Thus, Table 3 below provides an indication of the volumes of the biological component necessary to achieve given cell numbers based on continued culture:

**Table 3.**

| Clinical use | Cells/ml Yield required | 2.4.x10⁷ Day 9 of culture | 4.8 x 10⁷ Day 11 of culture | 6.0 x 10⁷ Day 12 of culture |
|---|---|---|---|---|
| Human (low esti mate 30%) | 3 x 10 ¹⁰ | 1250ml | 625ml | 500ml |
| Human (high estimate) | 7 x 10 ¹⁰ | 2916 ml | 1456ml | 1167ml |
| Human liver | 1 x 10 ¹¹ | 4167 ml | 2083ml | 1666ml |

As the novel methodology also gives rise to a novel biological component which will in turn be used in the chamber described in GB 0713595.7 for completeness details of:
a) The chamber (section 1.0);
b) The biological component (section 2.0)
c) The bio-artificial liver device, (section 3.0) and
d) It's use to perfuse a patient (section 4.0) are described below

### 1.0 EMPTY CHAMBER

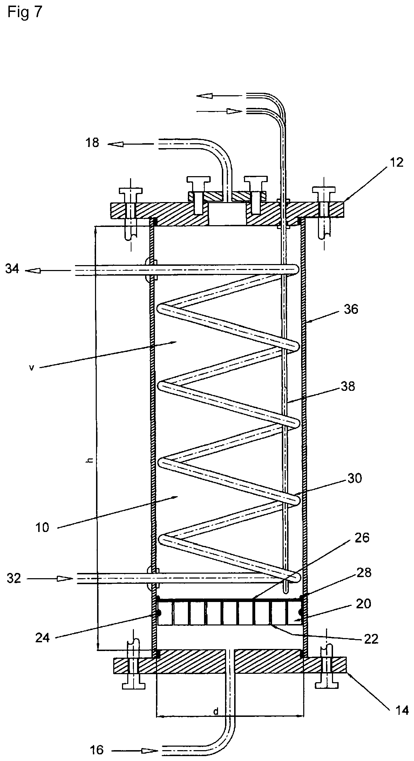

Referring to Fig 7, the chamber (10) is generally cylindrical in shape having a diameter (d), height (h) and a capacity or volume (v) appropriate to its function of liver perfusion. Typically this volume will be from 1 - 10 litres, more preferably 1 - 5 litres.

It is made of a material which can be sterilized (e.g. by autoclaving at 121°C at 1 bar), will withstand cryopreservation temperatures (-160°C) and can also withstand the rapid temperature change associated with these procedures. Additionally, the material should be compatible with the presence of those biological materials and solutions which it will contain, e.g. blood, plasma, saline, cryopreservatives, culture media etc.

It is a sealable unit comprising a walled cylindrical housing (36) enclosed by upper and lower plates (12, 14). As, in at least one mode of operation, it functions as a fluidised bed it comprises a fluidising inlet (16) in lower plate (14) and a fluidising outlet (18) in upper plate (12). These inlets/ outlets can be used to introduce solutions including: culture media, plasma and cryoprotectants. A fluid bed support (20) comprising a plurality of orderly positioned holes (22), which assist in controlling fluidisation, is held in place by an 0-ring (24) at the lowermost end of the chamber. Disposed over the fluid bed support is a mesh filter (26) which is held down by a wire ring (28).

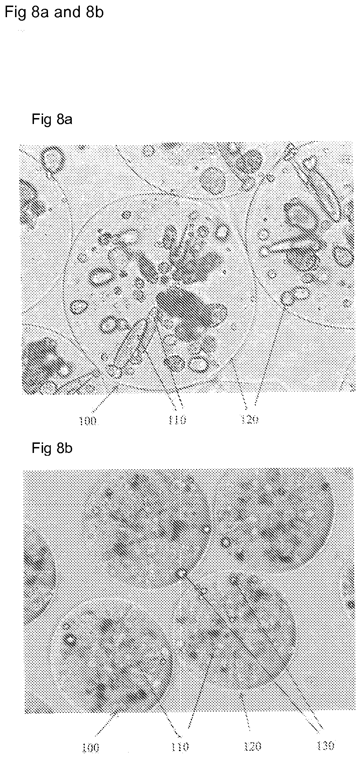

The filter serves to entrap alginate beads (120 - Fig 8) within the chamber during all stages of fluidisation and usage.

The chamber further comprises a fluid transport system (30) which enters the chamber at inlet (32) just above the fluid bed support (20) and exits the chamber at outlet (34) towards the upper plate (12). The fluid transport system takes the form of a tube which is preferably arranged helically, and in a regular pattern, around the inner wall (36) of the cylinder although other configurations are possible. The tube, which is semi-permeable to gases, will facilitate gas exchange (its primary function) to the fluidised biological component (100) (not shown for clarity). It can be made from silicone or any other suitable material, e.g. polymers which are gas permeable, non toxic, and can withstand the temperatures that will be experienced in operation. The material should also exhibit a degree of flex such that the tube can absorb the expansion which occurs when the contents of the chamber are frozen. The fluid transport system may additionally act as a heat exchange as fluids (liquids or gasses) are pumped there through.

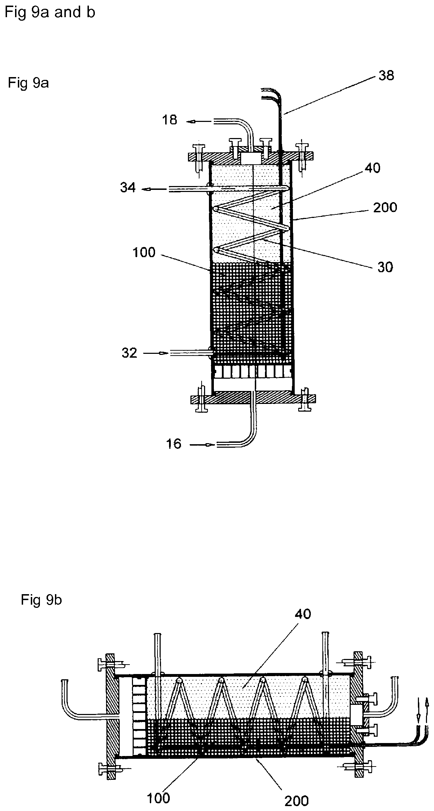

Finally, the chamber comprises one or a plurality of heat exchange elements (38) which in one embodiment extend down through upper plate (12) into the chamber to just above the fluid bed support (20). These elements are arranged to be substantially perpendicular to the plates (12, 14) and are arranged in a regular pattern (being substantially evenly distributed) within at least a part of the volume of the chamber so as to maximise even and efficient freezing and thawing whilst minimising their effect on fluidisation. To this end, in a preferred embodiment, the heat exchange elements (which in one embodiment comprise hollow rods made from or coated with titanium and alloys thereof to maximise heat exchange) are disposed along one length (h) of the chamber, such that when the chamber is placed horizontally (Fig 9b) (in contrast to its vertical fluidising, position (Fig 9a)) it is able to efficiently cool/ warm the biological component which is distributed favourably from a mass transfer perspective. It is important that the materials are non-toxic to the biological component and are not corroded by e.g. saline.

In an alternative arrangement the one or a plurality of heat exchange elements (38) may pass directly through the upper and lower plate in a straight path. Such an arrangement may simplify the pumping or pulling of a coolant fluid through the tubes. Indeed, in such an embodiment the tubes may have funnel shaped ends.

As, has been hinted at above with reference to the heat exchange elements (38) and fluid transport system (30), "scale up" brings with it issues of ensuring supply of e.g. oxygen and nutrients to the cells and exchange of e.g. proteins from the cells to e.g. blood / plasma. Accordingly, to assist in the exchange it is preferred that the cylinder has an aspect ratio (a), a = h /d, of from 10:1 to 1.3:1 and a capacity (v) of between 1 and 10 litres.

Whilst aimed at human liver cell lines in this instance, this design is generic for several proliferating mammalian epithelial cells and would be applicable to other biomass requirements. For more details on the favoured biological component see section 2.0.

Oxygenation can be provided in a number of ways including simple gas exchange through gas permeable membranes, hollow fibre oxygenators and /or utilising perfluorocarbon mixtures with high oxygen saturation capabilities

### 2.0 BIOLOGICAL COMPONENT

Referring to Figs 8a and 8b, the biological component (100) comprises a plurality of cells (110) encapsulated in alginate beads (120) of approximately 400µm in diameter. The alginate beads also comprise a plurality of density modifiers (130) in the form of glass beads.

The individual cells are encapsulated to achieve approximately 22 to >50 million cells per millilitre of beads at performance competence.

This 3-D alginate encapsulation system can of course be used for any proliferating epithelial cell lines so should functionally better cell lines emerge the technology will be equally effective. Development of alternative proliferating human cell lines, exhibiting a hepatocyte phenotype include differentiated hepatocyte cell lines from human embryonal, or cord blood stem cells.

### 3.0 BIO-ARTIFICIAL LIVER (BAL) DEVICE

The BAL device is illustrated in more detail in Figs 9a, and 9b. When the chamber (10) is filled with the biological component (100) it becomes, or at least has the potential to become, a bio-artificial liver (200) which may be used in an extracorporeal perfusion circuit (50).

To function as a bioartificial liver (200), the chamber (10) should comprise a volume (v) which is filled with sufficient biological component (100) and an appropriate solution (40), e.g. plasma, to function substantially, but temporarily, as a liver. (I.e. be fit for purpose). To this end, because the chamber functions as a fluidised bed, the biological component will be fluidised and thus the chamber capacity should be from about 1.2 to 2 times the volume of the bead volume added.

Obviously the bead volume will depend on the functionality of the liver cells encapsulated within the beads. A human liver can function at about 15%, and the BAL has been modelled to provide a 30% function (twice the minimal requirement).

Thus, it should comprise performance competent cell spheroids in 1-2% alginate beads, of a density that will allow fluidisation in human plasma. Performance competent cell spheroids will have been cultured for from 8-12 days and will contain anywhere from 22 to > 60 million cells per ml of beads.

In order to achieve this, a yield of ∼1 billion cells (1 x 10⁹) will be used to seed each 500-666ml of alginate beads, and multiples thereof, such that the chamber will contain between 3x10¹⁰ to 1x10¹¹ performance competent cells.

The alginate beads (120) will have a mean diameter of between 300 - 1200µm, preferably 400 µm, and will additionally contain glass beads (130) with a mean diameter of between 10 - 50µm in sufficient number to control the density for fluidisation to up to two times bed volume. The biological component (100) will be provided in an appropriate solution (40), either culture medium (during proliferation), isotonic fluid (pre use), plasma (pre-and during perfusion) or cryoprotectant (after proliferation when stored) depending on the phase of use.

In use the chamber will initially be fluidised at flow rates of between 50 ml/min and 1200ml/min to achieve a 1.2 to 2-fold bed expansion.

However, moving from the use of cell lines on a lab scale (70ml or less) to the development of a bio-artificial liver brings with it new challenges including the need to develop scalable methods and an understanding of how biological materials will function in the volumes necessary for use in liver perfusion on human subjects. It requires the multi-disciplinary expertise of mechanical engineering, mathematical modelling, biochemical engineering, materials science, theoretical physics, as well as medical expertise.

Significantly, the Applicant has now been able to demonstrate, on scales of up to 200ml, that this Fluidised bed chamber design of a BAL device, utilising 3-d spheroids of human liver-derived cell lines, can achieve metabolic performance akin to that found in vivo and in primary hepatocytes.

For example, sampling the reservoir of culture medium by collecting 1ml samples and analysing them has demonstrated:
- a glucose consumption of 4.98 µmoles glucose /million cells/day;
- similarly protein synthesis (to a human liver);
- secretion of albumin which demonstrate values in the same order of magnitude as that found in vivo, i.e. 12g/10¹² liver cells/day; and (utilising oxygen probes measuring dissolved oxygen)
- oxygen consumption of approximately 19µmoles O/min /mg protein.

Manifestly, any artificial liver must be capable of being manufactured and distributed efficiently and safely, using processes that comply with GMP requirements.

Applicant has additionally developed a means of storing the performance competent beads for up to 3 days at room temperature and pressure at a high bead to medium ratio by using oxygen saturated perfluorodecalin (an oxygen carrier with much higher saturation than aqueous solution).

This is important as it would allow a manufacturer to "defrost" a device containing performance competent cells and ship it direct to a user (who wouldn't need to defrost the device at the point of use) making it simpler to use and less prone to user error.

### 4.0 MODIFICATION FOR IN-LINE PATIENT USE

The final treatment phase will comprise connecting the bio-artificial liver device (200) (chamber (10) containing proliferated cells (100)) into a circuit (50) such that it is perfused with human plasma (40).

Thus, in use the BAL (200) will be connected to a patient such that plasma (40) enters the chamber (10) at, for example, inlet (16) and exits at, for example, outlet (18).

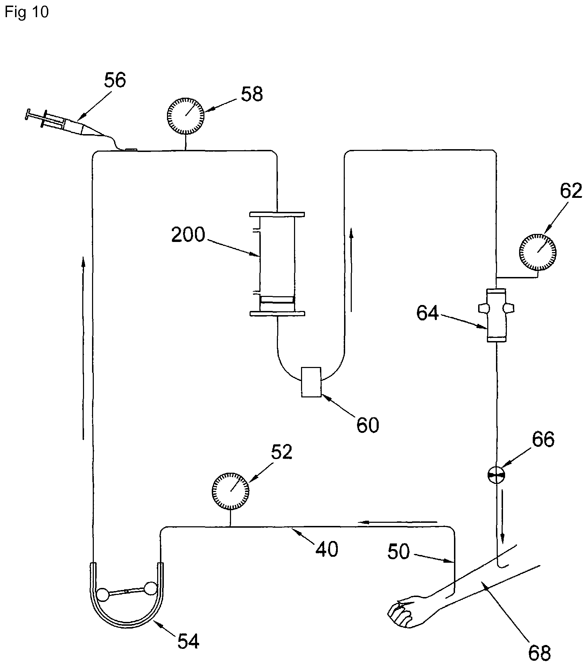

Referring to Fig 10, either whole blood is perfused in a circuit (50) or blood is removed from a patient (68) and the plasma (40) is separated from the blood cells and passes through a circuit (50), and through the BAL (200) before being reintroduced to the blood cells and returned to the patient. Typically, but not essentially, the blood will pass through an arterial pressure monitor (52), the plasma separated, and be driven by a pump (54) to the BAL (200) via a heparin pump (56) and a perfusion inflow pressure monitor (58). In the BAL, the plasma is treated allowing exchange of e.g. toxins from the plasma to the cells (110) and proteins from the cells to the plasma. On leaving the BAL, the plasma passes through a DNA removal cartridge (60), is reintroduced to the blood cells through a venous pressure monitor (62), air trap (64), and air detector (66) before being returned to the patient. Preferably the DNA removal cartridge comprises a microporous matrix with pores to achieve a molecular weight cut-off of 2 million Daltons.

The cartridge should also contain a DNA binding cassette comprised of immobilised Deoxyribonuclease I enzyme to cleave DNA into di- and tri-nucleotides.

In use the device is fluidised to up to a two-fold bed height in the presence of human plasma as part of the extracorporeal circuit (50) that will enable the chamber to function as a clinically useful biomass.

A normal liver contains ∼1 - 2 x 10¹¹ hepatocytes and ∼15% of normal liver mass is required to sustain life in otherwise well individuals (1.5 - 3.0 x10¹⁰).

Accordingly, the applicant's system has been developed with the aim of providing 30% of a normal liver cell mass. This cell mass must be contained in a practical perfusion system with diffusion parameters that allow e.g. adequate access of oxygen and nutrients during the initial proliferation phase, and which allows transfer of toxins in, and metabolites, detoxified products and proteins out of cells for return to a patient during inline use in extracorporeal perfusion. In this regard the Applicant's system, benefits from the use of uncoated alginate gel beads which minimise diffusion barriers. It also comprises a geometry suitable for use in a human extracorporeal circulation, by allowing adequate numbers of cells to be contained in a volume which can be feasibly perfused in a device for human use. If required, more than one chamber can be used in parallel.

Blood flow through the portal vein is 1200ml/min in man, and therefore, ideally the plasma should circulate through the chamber at a flow rate commensurate with in vivo conditions for the treatment regime. Previous work has indicated a flow rate of 400ml/min may be adequate for this step so flow rates ranging from 50-1200ml/min may be used.

## Claims

1. A biological component (100) for a bio-artificial liver, comprising:
i. a matrix forming agent comprising alginate beads,
ii. a plurality of cells encapsulated in the alginate beads, the cells comprising a proliferating human cell line exhibiting a hepatocyte phenotype, and
iii. one or more density modifiers encapsulated in the alginate beads, to control the density for fluidisation,
**characterized in that** the cells were grown in a fluidised bed reactor in a culture media supplemented with from 2-20% human plasma, and that, at performance competence, the cells are present in the matrix forming agent at a density of more than 3 x 10⁷ cells/ml, maintain a near cuboidal cell architecture, have a close cell to cell and cell to matrix organisation and excrete extracellular matrix proteins and a large repertoire of liver specific secreted proteins.

2. A biological component (100) as claimed in claim 1 wherein the cells are a Hep G2 cell line with or without genetic modification.

3. A biological component (100) as claimed in claim 1 wherein the alginate beads have a diameter of from 300-1200µm.

4. A method of growing a plurality of cells comprising a proliferating human cell line exhibiting a hepatocyte phenotype to performance competence in a matrix forming agent comprising alginate beads containing one or more density modifiers to control the density for fluidisation, such that they maintain a near cuboidal cell architecture, have a close cell to cell and cell to matrix organisation and excrete extracellular matrix proteins and a large repertoire of liver specific secreted proteins, **characterized in that** the cells are grown in a fluidised bed reactor in a culture media supplemented with from 2-20% human plasma.

5. A method as claimed in claim 4 where in the human plasma is leukocyte depleted fresh frozen human plasma.

6. A method as claimed in claim 5 wherein the fresh frozen human plasma contains heparin.

7. A method as claimed in claim 6 wherein the heparin is present in an amount of -40 units /ml.

8. A bio-artificial liver (200) comprising a chamber (10) filled with a biological component (100) comprising:
i. a matrix forming agent comprising alginate beads,
ii. a plurality of cells encapsulated in the alginate beads, the cells comprising a proliferating human cell line exhibiting a hepatocyte phenotype, and
iii. one or more density modifiers encapsulated in the alginate beads, to control the density for fluidisation,
**characterized in that** the cells were grown in a fluidised bed reactor in a culture media supplemented with from 2-20% human plasma, and that, at performance competence, the cells are present in the matrix forming agent at a density of at least 3 x 10⁷ cells/ml, maintain a near cuboidal cell architecture, have a close cell to cell and cell to matrix organisation and excrete extracellular matrix proteins and a large repertoire of liver specific secreted proteins.

9. A bio-artificial liver (200) as claimed in claim 8 comprising from 3x10¹⁰ to 1x10¹¹ competent cells in a volume of less than 2 litres.

10. A bio-artificial liver (200) as claimed in claim 9 comprising 3x10¹⁰ competent cells in a volume of less than 1 litre.

11. A bio-artificial liver (200) as claimed in claim 9 wherein the chamber comprises a fluid bed support (2), a fluidising inlet (16) and a fluidising outlet (18).

12. A scalable method for proliferating cells comprising a proliferating human cell line exhibiting a hepatocyte phenotype seeded in a matrix forming agent comprising alginate beads containing one or more density modifiers to control the density for fluidisation, comprising:
a. Placing the cells seeded in the matrix forming agent in a chamber having a fluidised bed, and
b. Growing them to performance competence such that they maintain a near cuboidal cell architecture, have a close cell to cell and cell to matrix organisation and excrete extracellular matrix proteins and a large repertoire of liver specific secreted proteins, in a culture media supplemented with from 2-20% human plasma.

13. A biological component according to any one of claims 1 to 3, wherein the one or more density modifiers are in the form of glass beads.

14. A method according to any one of claims 4 to 7, wherein the one or more density modifiers are in the form of glass beads.

15. A bio-artificial liver according to any one of claims 8 to 11, wherein the one or more density modifiers are in the form of glass beads.

16. A scalable method according to claim 12, wherein the one or more density modifiers are in the form of glass beads.

## Patentansprüche

1. Biologische Komponente (100) für eine bio-artifizielle Leber, die Folgendes umfasst:
i. einen Matrixbildner, der Alginatperlen umfasst,
ii. eine Vielzahl von in den Alginatperlen eingekapselten Zellen, wobei die Zellen eine sich teilende menschliche Zelllinie umfassen, die einen Hepatozyten-Phänotyp zeigt, und
iii. einen oder mehrere Dichtemodifizierer, die in den Alginatperlen eingekapselt sind, um die Dichte für eine Fluidisierung zu steuern,
**dadurch gekennzeichnet, dass** die Zellen in einem Wirbelschichtreaktor in einem mit von 2 - 20 % menschlichem Plasma ergänzten Kulturmedium gezüchtet wurden,
und dass die Zellen bei Leistungsfähigkeit im Matrixbildner in einer Dichte von mehr als 3 x 10⁷ Zellen/ml vorliegen, eine nahezu kubische Zellarchitektur beibehalten, eine enge Zell-zu-Zell und Zell-zu-Matrix Ordnung aufweisen und extrazelluläre Matrixproteine und ein großes Repertoire von leberspezifischen sekretierten Proteinen ausscheiden.

2. Biologische Komponente (100) wie in Anspruch 1 beansprucht, wobei die Zellen eine Hep G2-Zelllinie mit oder ohne genetische Modifikation darstellen.

3. Biologische Komponente (100) wie in Anspruch 1 beansprucht, wobei die Alginatperlen einen Durchmesser von 300 - 1200 µm aufweisen.

4. Verfahren zum Züchten einer Vielzahl von Zellen, die eine sich teilende menschliche Zelllinie umfassen, die einen Hepatozyten-Phänotyp zeigt, zur Leistungsfähigkeit in einem Matrixbildner, der Alginatperlen umfasst, die einen oder mehrere Dichtemodifizierer enthalten, um die Dichte für eine Fluidisierung zu steuern, so dass sie eine nahezu kubische Zellarchitektur beibehalten, eine enge Zell-zu-Zell und Zell-zu-Matrix Ordnung aufweisen und extrazelluläre Matrixproteine und ein großes Repertoire von leberspezifischen sekretierten Proteinen ausscheiden, **dadurch gekennzeichnet, dass** die Zellen in einem Wirbelschichtreaktor in einem mit von 2 - 20 % menschlichem Plasma ergänzten Kulturmedien gezüchtet werden.

5. Verfahren wie in Anspruch 4 beansprucht, wobei das menschliche Plasma ein an Leukozyten abgereichertes, frisches gefrorenes menschliches Plasma darstellt.

6. Verfahren wie in Anspruch 5 beansprucht, wobei das frische gefrorene menschliche Plasma Heparin enthält.

7. Verfahren wie in Anspruch 6 beansprucht, wobei das Heparin in einer Menge von ∼ 40 Einheiten/ml vorhanden ist.

8. Bio-artifizielle Leber (200) mit einer Kammer (10), die mit einer biologischen Komponente (100) gefüllt ist, die Folgendes umfasst:
i. einen Matrixbildner, der Alginatperlen umfasst,
ii. eine Vielzahl von in den Alginatprlen eingekapselten Zellen, wobei die Zellen eine sich teilende menschliche Zelllinie umfassen, die einen Hepatozyten-Phänotyp zeigt, und
iii. einen oder mehrere Dichtemodifizierer, die in den Alginatbeads eingekapselt sind, um die Dichte für eine Fluidisierung zu steuern,
**dadurch gekennzeichnet, dass** die Zellen in einem Wirbelschichtreaktor in einem mit von 2 - 20 % menschlichem Plasma ergänzten Kulturmedium gezüchtet werden und dass die Zellen bei Leistungsfähigkeit im Matrixbildner in einer Dichte von mindestens 3 x 10⁷ Zellen/ml vorliegen, eine nahezu kubische Zellarchitektur beibehalten, eine enge Zell-zu-Zell und Zell-zu-Matrix Ordnung aufweisen und extrazelluläre Matrixproteine und ein großes Repertoire von leberspezifischen sekretierten Proteinen ausscheiden.

9. Bio-artifizielle Leber (200) wie in Anspruch 8 beansprucht, die von 3 x 10¹⁰ bis 1 x 10¹¹ kompetente Zellen in einem Volumen von weniger als 2 Litern umfasst.

10. Bio-artifizielle Leber (200) wie in Anspruch 9 beansprucht, die 3 x 10¹⁰ kompetente Zellen in einem Volumen von weniger als 1 Liter umfasst.

11. Bio-artifizielle Leber (200) wie in Anspruch 9 beansprucht, wobei die Kammer einen Wirbelschichtträger (2), einen fluidisierenden Einlass (16) und einen fluidisierenden Auslass (18) umfasst.

12. Skalierbares Verfahren für sich teilende Zellen, die eine sich teilende menschliche Zelllinie umfassen, die einen Hepatozyten-Phänotyp zeigt, welche in einem Matrixbildner ausgesät ist, der Alginatperlen umfasst, die einen oder mehrere Dichtemodifizierer enthält, um die Dichte für eine Fluidisierung zu steuern, wobei das Verfahren Folgendes umfasst:
a. Anordnen der in dem Matrixbildner ausgesäten Zellen in einer Kammer, die eine Wirbelschicht aufweist, und
b. Züchten dieser zu Leistungsfähigkeit, so dass sie eine nahezu kubische Zellarchitektur beibehalten, eine enge Zell-zu-Zell und Zell-zu-Matrix Ordnung aufweisen und extrazelluläre Matrixproteine und ein großes Repertoire von leberspezifischen sekretierten Proteinen ausscheiden, in einem mit von 2 - 20 % menschlichem Plasma ergänzten Kulturmedium.

13. Biologische Komponente nach einem der Ansprüche 1 bis 3, wobei der eine oder die mehreren Dichtemodifizierer in Form von Glasperlen vorliegen.

14. Verfahren nach einem der Ansprüche 4 bis 7, wobei der eine oder die mehreren Dichtemodifizierer in Form von Glasperlen vorliegen.

15. Bio-artifizielle Leber nach einem der Ansprüche 8 bis 11, wobei der eine oder die mehreren Dichtemodifizierer in Form von Glasperlen vorliegen.

16. Skalierbares Verfahren nach Anspruch 12, wobei der eine oder die mehreren Dichtemodifizierer in Form von Glasperlen vorliegen.

## Revendications

1. Composant biologique (100) pour un foie bio-artificiel, comprenant :
i. un agent de formation de matrice comprenant des perles d'alginate,
ii. une pluralité de cellules encapsulées dans les perles d'alginate, les cellules comprenant une lignée cellulaire humaine proliférant présentant un phénotype d'hépatocyte et
iii. un ou plusieurs modificateurs de densité encapsulés dans les billes d'alginate pour contrôler la densité de fluidisation,
**caractérisé en ce que** les cellules ont été cultivées dans un réacteur à lit fluidisé dans un milieu de culture complémenté avec 2-20 % de plasma humain, et que, dans la compétence de la performance, les cellules sont présentes dans l'agent de formation de matrice, à une densité de plus de 3x10⁷ cellules/ml, maintiennent une architecture de cellules presque cuboïdes, ont une étroite organisation cellule à cellule et cellule à matrice et excrète des protéines de matrice extracellulaire et un vaste répertoire de protéines sécrétées spécifiques du foie.

2. Composant biologique (100) selon la revendication 1, dans lequel les cellules sont une lignée cellulaire Hep G2 avec ou sans modification génétique.

3. Composant biologique (100) selon la revendication 1, dans lequel les perles d'alginate ont un diamètre de 300 à 1200 µm.

4. Procédé de croissance d'une pluralité de cellules comprenant une lignée cellulaire humaine proliférant présentant un phénotype d'hépatocyte à une compétence de performance dans un agent de formation de matrice comprenant des perles d'alginate contenant un ou plusieurs modificateurs de densité pour contrôler la densité de fluidisation de telle sorte qu'ils maintiennent une architecture de cellule presque cuboïde ont une étroite organisation cellule à cellule et cellule à matrice et excrètent des protéines de matrice extracellulaire et un grand répertoire de protéines sécrétées spécifiques du foie, **caractérisé en ce que** les cellules sont cultivées dans un réacteur à lit fluidisé dans un milieu de culture complété par de 2 à 20 % de plasma humain.

5. Procédé selon la revendication 4, dans lequel, dans le plasma humain, se trouve un plasma humain congelé frais appauvri en leucocytes.

6. Procédé selon la revendication 5, dans lequel le plasma humain frais congelé contient de l'héparine.

7. Procédé selon la revendication 6, dans lequel l'héparine est présente en une quantité de -40 unités / ml.

8. Foie bio-artificiel (200) comprenant une chambre (10) remplie d'un composant biologique (100) comprenant :
i. un agent de formation de matrice comprenant des perles d'alginate,
ii. une pluralité de cellules encapsulées dans les perles d'alginate, les cellules comprenant une lignée cellulaire humaine proliférant présentant un phénotype hépatocytaire, et
iii. un ou plusieurs modificateurs de densité encapsulés dans les perles d'alginate, pour contrôler la densité de fluidisation,
**caractérisé en ce que** les cellules sont cultivées dans un réacteur à lit fluidisé dans un milieu de culture additionné de 2 à 20 % de plasma humain, et que, à leur niveau de performance, les cellules sont présentes dans l'agent formant la matrice à une densité d'au moins 3x10⁷ cellules/ml, maintiennent une architecture de cellule presque conoïde, ont une étroite organisation cellule à cellule et cellule à matrice et excrète des protéines de matrice extracellulaire et un grand répertoire de protéines sécrétées spécifiques du foie.

9. Foie bio-artificiel (200) selon la revendication 8, comprenant de 3x10¹⁰ à 1x10¹¹ cellules compétentes dans un volume inférieur à 2 litres.

10. Foie bio-artificiel (200) selon la revendication 9 comprenant 3x10¹⁰ cellules compétentes dans un volume inférieur à 1 litre.

11. Foie bio-artificiel (200) selon la revendication 9, dans lequel la chambre comprend un support de lit fluidisé (2), une entrée de fluidisation (16) et une sortie de fluidisation (18).

12. Procédé évolutif pour proliférer des cellules comprenant une lignée cellulaire humaine proliférant présentant un phénotype d'hépatocyte ensemencé dans un agent formant une matrice comprenant des billes d'alginate contenant un ou plusieurs modificateurs de densité pour contrôler la densité de fluidisation, comprenant :
a. placer les cellules ensemencées dans l'agent formant la matrice dans une chambre ayant un lit fluidisé, et
b. en les cultivant à des compétences de performance telles qu'elles maintiennent une architecture de cellule presque cuboïde, ont une étroite organisation cellule à cellule et cellule à matrice et excrètent des protéines de matrice extracellulaire et un grand répertoire de protéines sécrétées spécifiques de foie, dans un milieu de culture supplémenté de 2- 20 % de plasma humain.

13. Composant biologique selon n'importe laquelle des revendications 1 à 3, dans lequel le ou les agents modificateurs de densité sont sous la forme de billes de verre.

14. Procédé selon l'une quelconque des revendications 4 à 7, dans lequel le ou les modificateurs de densité sont sous forme de perles de verre.

15. Foie bio-artificiel selon l'une quelconque des revendications 8 à 11, dans lequel le ou les agents modificateurs de densité sont sous forme de perles de verre.

16. Procédé évolutif selon la revendication 12, dans lequel le ou les modificateurs de densité sont sous forme de perles de verre.
